# EUROPEAN PATENT SPECIFICATION

(11) **EP 3 617 188 B1**
(45) Date of publication and mention of the grant of the patent: **29.05.2024**
(21) Application number: 18792235.6
(22) Date of filing: 27.04.2018
(51) Int. Cl.: C07C 319/20, C07C 319/28, C07C 323/58, B04B 1/20

(54) **METHIONINE PRODUCTION METHOD AND PRODUCTION EQUIPMENT**
METHIONINHERSTELLUNGSVERFAHREN UND HERSTELLUNGSAUSRÜSTUNG
PROCÉDÉ DE PRODUCTION DE MÉTHIONINE ET ÉQUIPEMENT DE PRODUCTION

(30) Priority: 27.04.2017 JP 2017087750
(43) Date of publication of application: 04.03.2020
(73) Proprietor: SUMITOMO CHEMICAL COMPANY, LIMITED, Tokyo 103-6020 (JP)
(72) Inventor: OMOTO, Norihito, Ehime 792-8521 (JP); SATOH, Yoshitaka, Ehime 792-8521 (JP); MORIKAWA, Masayuki, Ehime 792-8521 (JP); KOIZUMI, Yoshiyuki, Ehime 792-8521 (JP); YAMASHIRO, Naoya, Ehime 792-8521 (JP); KATAGAMI, Ryousuke, Ehime 792-8521 (JP)
(74) Representative: J A Kemp LLP
(86) International application number: PCT/JP2018/017221
(87) International publication number: WO 2018/199296

(56) References cited:
- JP-A- S 511 415
- JP-A- H04 169 570
- JP-A- 2009 292 796
- JP-A- 2010 111 640
- JP-A- 2012 201 672
- US-A1- 2010 121 103

## Description

### TECHNICAL FIELD

This application claims priority to and the benefit of Japanese Patent Application No. 2017-087750 filed April 27, 2017.

The present invention is related to a method for producing methionine by hydrolysis of 5-[2-(methylthio)ethyl] imidazoline-2,4-dione.

### BACKGROUND ART

Methionine is one kind of an essential amino acid that cannot be synthesized in a body in an animal, and is widely used as a feed additive for animal, and also is industrially produced by a chemical plant.

As an example of production method of methionine, a method by hydrolysis of 5-[2-(methylthio)ethyl]imidazoline-2,4-dione is known (for example, see Patent document 1 and Patent document 2). In the method, methionine is precipitated by hydrolyzing 5-[2-(methylthio)ethyl]imidazolidine-2,4-dione, followed by introducing carbon dioxide, and a first crystallization step for separating methionine and mother liquor is conducted, and a useful component such as methionine dissolved in the mother liquor obtained in the first crystallization step is precipitated, and a second crystallization step for separating the useful component and the mother liquor is conducted.

### CITATION LIST

### PATENT DOCUMENT

Patent Document 1: JP 5307512 B Patent Document 2: US 2010/121103 A1 and JP 2010 111641 A

### SUMMARY OF THE INVENTION

### (PROBLEMS TO BE SOLVED BY INVENTION)

In the second crystallization step, when the precipitates as the useful component are separated from the mother liquor, a part of the precipitates may float in the mother liquor without settling, or may float on the surface of the mother liquor, and as a result, the useful component may remain in the mother liquor. Since the loss of useful components affects the production cost, it is important to reduce the loss.

An object of the present invention is to efficiently recover active useful component such as methionine dissolved in a mother liquor after a separation of methionine in the first crystallization step in a method for producing methionine, thereby reducing production loss.

### (MEANS TO SOLVE PROBLEMS)

The present inventors have intensively studied, and as a result, found that when a useful component such as methionine dissolved in the mother liquor after separation of methionine in the first crystallization step is precipitated and collected by a solid-liquid separation, the solid-liquid separation is conducted by a centrifuge and a filter, so that the useful component can be recovered efficiently and the production loss is reduced.

The present invention encompassed the following embodiments.
[1] A method for producing methionine comprising the following steps (1) to (3):
   (1) a reaction step: a step of hydrolyzing 5-[2-(methylthio)ethyl]imidazolidine-2,4-dione in the presence of an alkali compound to obtain a reaction solution containing an alkali salt of methionine,
   (2) a first crystallizing step: a step of introducing carbon dioxide into the reaction solution and precipitating methionine from the reaction solution to obtain the first crystal of methionine and the first crystal mother liquor, and
   (3) a second crystallizing step: a step in which after obtaining a slurry which contains precipitates containing the second crystals of methionine precipitated by adding alcohol to the first crystallization mother liquor and then introducing carbon dioxide, (a) the slurry is subjected to centrifugation to separate the second crystals of methionine and the second crystal mother liquor by a solid-liquid separation at a centrifugal force 100 times or more to 4000 times or less gravity, and (b) the precipitate remaining in the obtained second crystal mother liquor is separated with a filter (hereinafter, referred to as "Method for producing methionine of the present invention").
[2] The method according to [1] wherein the precipitate is dissolved in the first crystal mother liquid, and added to the reaction solution.
[3] The method according to [1] or [2] wherein a portion of the second crystal mother liquor is recycled to the second crystallization step.
[4] The method according to any one of [1] to [3] wherein the material of the filter is polyphenylene sulfide or polypropylene.
[5] A production apparatus comprising
   a reaction apparatus in which 5-[2-(methylthio)ethyl]imidazolidine-2,4-dione is hydrolyzed in the presence of an alkali compound to obtain a reaction solution containing an alkali salt of methionine,
   a first crystallization apparatus in which carbon dioxide is introduced into the reaction solution to precipitate methionine from the reaction solution, and
   a second crystallization apparatus in which alcohol is added to the first crystallization mother liquor which is obtained after separating methionine obtained in the first crystallization apparatus and carbon dioxide is introduced to obtain a slurry containing the precipitated precipitates,
   a centrifugal separator for solid-liquid separation of the slurry containing the precipitate at a centrifugal force 100 times or more to 4000 times or less gravity, and
   a filter for separating the precipitates remained in the second crystal mother liquid obtained by a solid-liquid separation with the centrifuge,
(hereinafter, referred to as "Production apparatus of the present invention").

According to the method for producing methionine of the present invention, in the second crystallization step, the slurry containing the precipitates is subjected to a solid-liquid separation using a centrifuge, and then the precipitates remained in the second crystal mother liquor separated by the solid-liquid separation is separated by a filter(s), thereby precipitates that have been floated on the liquid or suspended in the liquid that could not be separated by the centrifuge can be recovered, and thereby a loss during the production of methionine can be reduced.

### [Brief Description of Drawings]

[Figure 1] Figure 1 indicates an explanatory drawing of the centrifuge periphery used by the production method of methionine according to embodiment of the production method described herein.
[Figure 2] Figure 2 indicates a flowchart before and after the second crystallization step in the method for producing methionine according to the embodiment described in the present application.
[Figure 3] Figure 3 indicates a schematic block diagram of the filtration performance test apparatus used for the filtration test of the second crystal mother liquid.
[Figure 4] Figure 4 indicates a graph showing the relation between filtration period and filtration amount for each filter.

### MODE FOR CARRYING OUT THE INVENTION

A method for producing methionine is explained, however, the embodiment described below exemplifies a method for producing methionine for explanation, and the method for producing methionine is not limited to the followings.

In the method for producing methionine as used herein, 5-[2-(methylthio)ethyl]imidazolidine-2,4-dione is used as a raw material, and is hydrolyzed in the presence of an alkali compound to obtain a reaction solution containing methionine as an alkali salt [(1) reaction step].

The starting 5-[2-(methylthio)ethyl]imidazolidine-2,4-dione can be obtained, for example, by reacting 2-hydroxy-4-methylthiobutanenitrile with ammonia and carbon dioxide or with ammonium carbonate.

Examples of the alkali compound include potassium hydroxide, sodium hydroxide, potassium carbonate, potassium hydrogen carbonate and the like, and two or more of them can be used as necessary. The amount of the alkali compound used is usually 2 to 10 moles, preferably 3 to 6 moles as potassium or sodium . per mole of 5-[2-(methylthio)ethyl]imidazolidine-2,4-dione. Also the amount of water used is usually 2 to 20 parts by weight per 1 part by weight of 5-[2-(methylthio)ethyl]imidazolidine-2,4-dione.

The hydrolysis reaction conducted in the reaction step is a stirring type or non-stirring type, and is conducted in a continuous type or batch type reaction tank.

This hydrolysis reaction is preferably carried out by heating to about 150 to 200°C under pressurized pressure of about 0.5 to 1 MPa as a gauge pressure. The reaction period is usually 10 minutes to 24 hours.

In order to extract methionine from the hydrolysis reaction solution thus obtained, carbon dioxide is introduced into the reaction solution for crystallization, and the resulting slurry is separated into a precipitate and a mother liquor by filtration, decantation, or the like to obtain the precipitated methionine as the first crystal [(2) First crystallization step]. As used herein, the term of "first crystal mother liquid" refers to a mother liquid obtained by separating methionine in the first crystallization step.

Carbon dioxide is absorbed into the reaction solution by introducing carbon dioxide, and the alkali salt of methionine is precipitated as free methionine.

The introduction of carbon dioxide is preferably conducted under pressurized pressure of usually 0.1 to 1 MPa, preferably 0.2 to 0.5 MPa as a gauge pressure.

The crystallization temperature is usually 0 to 50°C, preferably 10 to 30°C. The crystallization period may be a time from when the hydrolysis reaction solution is saturated with carbon dioxide until methionine is sufficiently precipitated, but is usually 10 minutes to 24 hours.

The separated methionine may be made into a product by drying, after washing or pH adjustment, if necessary. The drying is preferably carried out by heating to about 50 to 160°C under a slightly reduced pressure, atmospheric pressure, or pressurized pressure, and the drying period is usually 10 minutes to 24 hours.

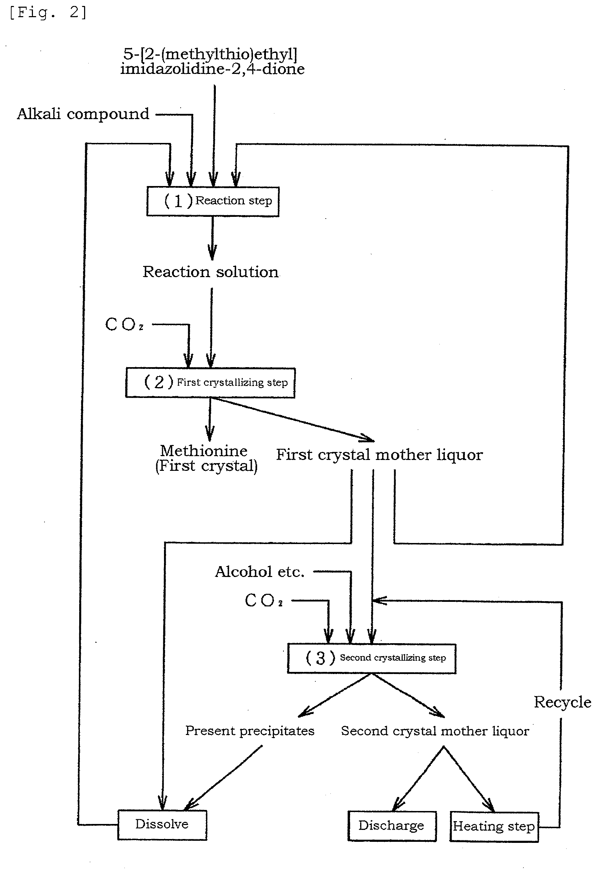

In Fig.2, the flowchart before and behind the second crystallization step in the production method of methionine according to embodiment of the present invention is shown. In the first crystal mother liquid, methionine corresponding to the solubility is remained, and a recyclable compound is contained as the alkali compound. As used herein, a useful component refers to methionine and the compound which can be recycled as the alkali compound.

Examples of the compound that can be recycled as the alkali compound include potassium hydrogen carbonate, potassium carbonate, potassium hydroxide, and potassium bonded to an organic substance (methionine potassium, potassium formate, potassium acetate, etc.). The first crystal mother liquor contains these useful components, and it is desirable to recycle the first crystal mother liquor for the hydrolysis reaction in step (1). On the other hand, because of impurities contained in the raw materials or the side reaction in hydrolysis, for example, amino acids other than methionine such as glycine and alanine, and coloring components are also contained, and these impurities may be brought into the hydrolysis reaction by recycling. Therefore, it is necessary to recycle the first crystal mother liquor in a range that does not accumulate impurities, not the whole amount, and the ratio is usually 50 to 90% by weight, preferably 60 to 85% by weight as opposed to the total amount of the first crystal mother liquid.

Although not shown in Fig.2, it is preferable to recycle the first crystal mother liquor by concentrating the first crystal mother liquor and using this concentrated solution as a recycle solution. By this concentration, carbon dioxide can be distilled off from the first crystal mother liquor, and a recycle liquid having an improved basicity and advantageous for the hydrolysis reaction can be obtained. Also, by conducting this concentration at a high temperature of 100 to 140°C, the reaction (2KHCO₃ → K ₂CO₃ + H₂O + CO₂) in which potassium hydrogen carbonate in the first crystal mother liquor is converted to potassium carbonate was promoted, and the basicity was further enhanced, and thereby a recycle liquid being advantageous to the hydrolysis reaction can be obtained. This concentration can be conducted under atmospheric pressure, reduced pressure, or pressurized pressure. In order to conduct the concentration at a high temperature as described above, it is effective to adopt a pressurized condition. The concentration rate is usually 1.2 to 4 times, preferably 1.5 to 3.5 times, where the concentration rate is the ratio of the liquid weight before concentration divided by the liquid weight after concentration ({liquid weight before concentration} / {liquid weight after concentration}), and so on.

A part or all of the first crystal mother liquor (concentrated mother liquor) that has not been recycled is subjected to a crystallization in order to precipitate the useful component dissolved in the first crystal mother liquor and recover it as a precipitate. In the present embodiment, this crystallization is conducted by adding alcohol to the first crystal mother liquid and introducing carbon dioxide, and separating the obtained slurry into a precipitate and a second crystal mother liquid, thereby recovering the useful component dissolved in the first mother liquor is recovered [(3) second crystallization step]. Hereinafter, the precipitate that is precipitated by adding alcohol to the first crystal mother liquor and followed by introducing carbon dioxide is referred to as the term of "the present precipitate". Also as used herein, the term of "second crystal mother liquor" refers to a mother liquor obtained by separating the present precipitate in the second crystallization step, and represents both a mother liquor obtained by solid-liquid separation using a centrifuge and a mother liquor obtained by separating the present precipitate using a filter, which is described below. In addition, the whole quantity can also be subjected to this crystallization, without recycling the concentrated first crystal mother liquid.

Although not shown in Fig.2, it is preferable that the first crystallization mother liquor being subjected to the second crystallization step is concentrated in the same manner as the first crystallization mother liquor to be recycled. By this concentration, the recovery rate of the present precipitate can be increased. This concentration can be conducted under the same conditions as the concentration of the first crystal mother liquor to be recycled, and after the whole amount of the first crystal mother liquor is concentrated, it may be divided into a recycle step and a second crystallization step.

In the concentration of the first crystal mother liquor, the basicity in the mother liquor increases, and the free methionine converted in the first crystallization step returns to the alkali salt of methionine. Therefore, also in the second crystallization step, the alkali salt of methionine is again converted to free methionine by introducing carbon dioxide into the mixed solution of the first crystal mother liquor and the alcohol after concentration.

Also it is preferable to heat-treat after concentration of the first crystal mother liquor, and a regeneration of methionine is promoted by hydrolysis of methionine dipeptide (dehydrated condensate of two methionine molecules) contained therein. This heat treatment is preferably performed at a temperature of about 140 to 180°C under pressurized pressure of about 0.5 to 2.0 MPa as a gauge pressure, and the heat treatment period is usually 10 minutes to 24 hours.

As the alcohol added to the first crystal mother liquor, an alkyl alcohol having an alkyl group having 1 to 5 carbon atoms is usually used, among them, one or two or more of alcohols which are miscible with water at an arbitrary ratio, such as methyl alcohol, ethyl alcohol, n-propyl alcohol, isopropyl alcohol, t-butyl is preferred, and isopropyl alcohol is particularly preferred. The amount of alcohol used is usually 0.05 to 5 times by weight, preferably 0.1 to 2 times by weight, to the first crystal mother liquid to be subjected to crystallization. The operation of adding alcohol to the first crystal mother liquor may be conducted before the introduction of carbon dioxide, or may be conducted simultaneously with the introduction of carbon dioxide.

The introduction of carbon dioxide in the second crystallization step is preferably carried out under pressurized pressure of usually 0.1 to 1.0 MPa as a gauge pressure, preferably 0.2 to 0.5 MPa, similarly to the first crystallization step. The crystallization temperature is usually 0 to 50°C, preferably 5 to 20°C. The crystallization period may be a time until the first crystal mother liquor to which alcohol has been added is saturated with carbon dioxide and the present precipitates are sufficiently precipitated, but is usually 10 minutes to 24 hours.

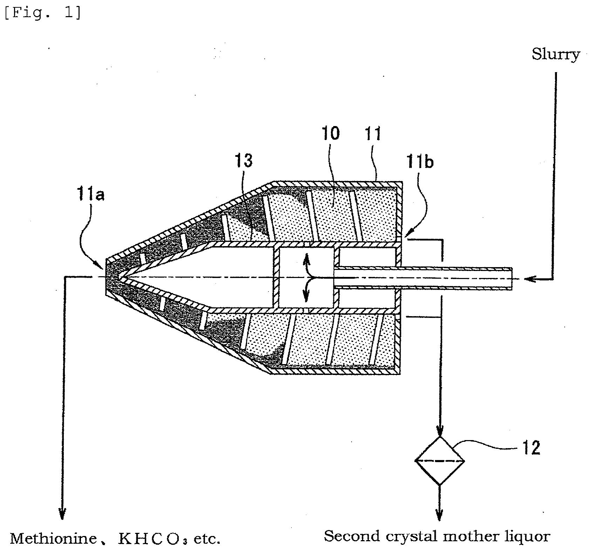

Fig. 1 is an explanatory view of the periphery of a centrifuge that continuously separates a slurry containing the present precipitates into the present precipitate and the second crystal mother liquor in the second crystallization step. Fig. 1 is a schematic sectional view of a decanter 10 that is one of horizontal centrifuges. The decanter 10 is configured to provide a rotating drum 11 that rotates about the center line depicted in Fig. 1 and a screw 13 that can have a rotational speed difference from the rotating drum 11, in the present embodiment, the slurry is continuously flowed in the vicinity of the center line, and the present precipitates are flown out from the first outlet 11a on the left side of the paper surface of Fig.1, and the second crystal mother liquor flows out from the second outlet 11b on the right side of the on-paper rotating cylinder in Fig 1 to separate the present precipitates and the second crystal mother liquor. Here the type of the centrifuge is not particularly limited, but a continuous type is preferable when expanding the production scale. A vertical centrifuge can also be used, and a horizontal or cylindrical type can also be used.

The centrifugal force in the solid-liquid separation by the centrifuge is 100 times or more and 4000 times or less to gravity. That is, the values of centrifugal effect: Z = mrω² / mg, m: mass [kg], r: turning radius [m], ω: angular velocity [rad / s], g: gravitational acceleration [m / s²] are preferably 100 or more or 4000 or less.

In the present embodiment, the present precipitates remained in the second crystal mother liquor is separated using the filter 12 without being separated by solid-liquid separation using a centrifuge. As described above, the slurry containing the present precipitates is separated into the present precipitates and the second crystal mother liquid by centrifugation. At this time, a part of the precipitates are remained in the second crystal mother liquid by floating in the liquid without settling or floating on the liquid surface. Therefore, in the present embodiment, a filter 12 for separating the present precipitate remained in the second crystal mother liquid is provided.

The filter material is preferably polypropylene or polyphenylene sulfide, more preferably polyphenylene sulfide. This is because, since the slurry is alkaline, when the liquid is filtered, the life of the filter can be extended while ensuring the performance of the filter. Thereby, the maintenance work for filter replacement can be reduced, and the cost associated with filter replacement can be reduced. Also, although not shown in the figure, the operation of the filter is, for example, by providing filters in parallel and replacing the filter through which the liquid passes at regular intervals, so that when the liquid is not passing through, the cakes collected with a filter can be recovered, for example, with the first crystal mother liquor. Further, it is possible to operate with one unit by using one filter and temporarily storing the mother liquor in a tank or the like.

The filter may be of any size that can collect the precipitates contained in the second crystal mother liquor. A filter capable of collecting precipitates having a particle size of 0.1 to 1,000 µm, preferably 1 to 500 µm.

As shown in Fig. 2, the recovered present precipitates are recycled to the hydrolysis reaction in step (1). At this time, it is desirable that the present precipitates are dissolved in the first crystal mother liquid for recycling, and added to the reaction solution.

The useful component is still dissolved and contained in the second crystal mother liquor after separating the present precipitates by the filter. As shown in Fig. 2, in the present embodiment, it is desirable that the second crystal mother liquor is concentrated in order to further recover the useful component from the second crystal mother liquor, and then a part thereof is heat-treated [heating step], and the solution after the heat-treat is recycled to the second crystallization method (preferably mixing with the concentrates of the first crystal mother liquid) to recover the useful components.

Also, the second crystal mother liquor is preferably not recycled wholly, but partially recycled and the rest discharged. This is because the second crystal mother liquor contains impurities as well as the first crystal mother liquor. By recycling a part of the second crystal mother liquor to the second crystallization method and discharging the rest, the adverse effects on the reaction step and the crystallization step due to the accumulation of impurities can be reduced. The loss of the present precipitates remained in the second crystal mother liquor leads to increase in production cost, and reducing the loss becomes more and more important in the method of recycling a part of the second crystal mother liquor and discharging the rest.

By concentrating the second crystal mother liquor, the recovery rate of methionine can be increased, and further, the alcohol added in the second crystallization step can be distilled off.

Also, the heat treatment after concentration promotes the regeneration of methionine by hydrolysis of the methionine dipeptide contained therein. This heat treatment is conducted at a temperature of 150 to 200°C, preferably 160 to 180°C under pressurized pressure of about 0.5 to 2 MPa as a gauge pressure. The heat treatment period is preferably 0.3 to 10 hours, more preferably 0.5 to 5 hours.

This heat treatment is preferably carried out until the ratio of methionine dipeptide to methionine is preferably 5 to 50% by weight, more preferably 5 to 40% by weight.

### [EXAMPLES]

Examples of the present invention are shown below, but the present invention is not limited thereto.

### [Example 1]

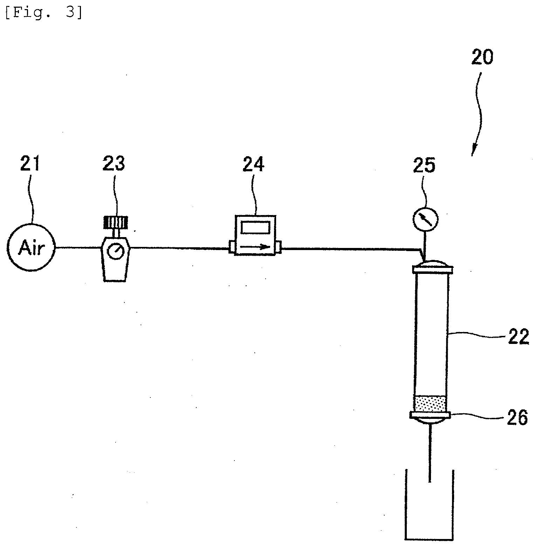

In Fig.3, the schematic block diagram of the filtration performance test device 20 used for the filtration test of the second crystal mother liquid containing the present precipitates is shown. The filtration performance test device 20 has on the bottom side of the filter 22 a filtration test filter 26 made of polypropylene with an average pore diameter of 5 um and a ventilation rate of 5-15 L / (dm² × min) @ 20 mm W. C. made of polypropylene as the same as used in the second crystallization method. Here, the air flow rate is 5-15 L / (dm² × min) @ 20 mmW. C. means that when air is passed under the condition of 200 Pa, the amount of air passing through the filter per minute is 5 to 15 L per unit area (1 dm²) of the filter. A second crystal mother liquor containing the present precipitates is supplied into a filter in which the filter 26 for filtration test is set, and compressed air at a predetermined flow rate is supplied from the compressed air supply device 21. At this time, the pressure of the compressed air is adjusted by the pressure reducing valve 23, and the pressure value is measured by the pressure gauge 25. Also the flow rate of the supplied compressed air is measured by the flow meter 24. The second crystal mother liquor that has passed through the filter 26 for filtration test is added dropwise under the filter 22. The weight and liquid content of the present precipitates remained on the filter 26 for filtration test are measured. The liquid content is a value measured by drying using an infrared moisture meter at a drying temperature of 80°C until the change in liquid content for 30 seconds is 0.05% by weight or less.

After obtaining the slurry containing the precipitates which precipitates by adding alcohol to the first crystal mother liquor and followed by introducing carbon dioxide, the slurry was subjected to solid-liquid separation with a centrifuge to obtain a second crystal mother liquor. Two hundreds (200) g of the second crystal mother liquor was supplied to the filter 22, and the filtration pressure was 0.3 MPa as a gauge pressure, and filtration was conducted for 30 minutes. The weight of the present precipitates on the filter 26 for the filtration device was 7.4 g, and the liquid content was 23.2% by weight. The weight of the precipitates recovered from the second crystal mother liquor, determined by calculation from these values, was 5.68 g. Also, the change in the appearance of the second crystal mother liquor before and after filtration was confirmed visually. The appearance of the second crystal mother liquor was cloudy before filtration, but was transparent after filtration.

### [Example 2]

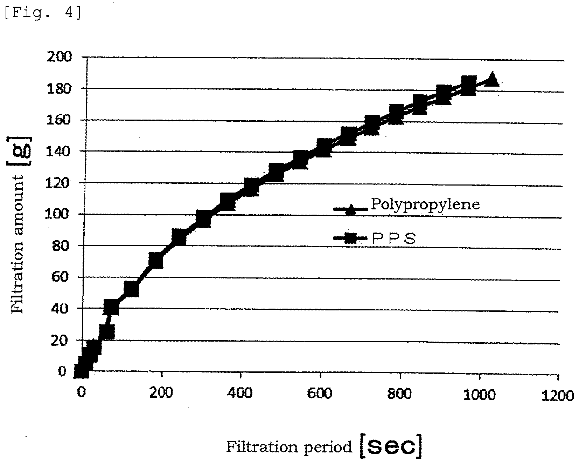

FIG. 4 shows a graph of the relationship between the filtration period and the filtration amount when filtered using two filters, Table 1 shows the main specifications of the filter used in the experiment, and Table 2 shows the experiment result. Also in this example, the filtration performance test device used in Example 1 was used.

**[Table 1]**

| Filter No. | Material | Pore diameter µm-50% | Ventilation rate L/(dm² x min)@20mmW.C. |
|---|---|---|---|
| 1 | polypropylene | 5 | 5-15 |
| 2 | polyphenylene sulfide (PPS) | 2.9 | 6-10 |

Here the pore diameter represents the average (um) of the pore diameter of the filter. Also the ventilation rate represents the amount (L) of air that passes through the filter per minute per unit area (1 dm²) of the filter when air is passed under the condition of 200 Pa.

**[Table 2]**

| Filter No. | Amount of second crystal mother liquor | Concentration of Precipitates | Filtration pressure |
|---|---|---|---|
| | g | % by weight | MPaG |
| 1 | 199.6 | 2.78 | 0.2 |
| 2 | 200.4 | 2.74 | 0.2 |

Filter No. 1 is made of polypropylene, and other specifications are as shown in Table 2. In the display of FIG. 4, the material of the filter is shown for easy understanding. Filter No. 2 is made of polyphenylene sulfide (PPS), and other specifications and specifications at the time of the experiment are as shown in Table 2 and [0037].

From FIG. 4, it was found that use of each material as the filter caused no problem by appropriately selecting the air flow rate and the pore diameter.

### Explanation of symbol

- 10: Decanta
- 11: Rotating body
- 12: Filter
- 13: Screw
- 20: Filtration performance test apparatus
- 21: Compressed air supply device
- 22: Filter
- 23: Pressure reducing valve
- 24: Flowmeter
- 25: Pressure gauge
- 26: Filtration test filter

## Claims

1. A method for producing methionine comprising the following steps (1) to (3):
(1) a reaction step: a step of hydrolyzing 5-[2-(methylthio)ethyl]imidazolidine-2,4-dione in the presence of an alkali compound to obtain a reaction solution containing an alkali salt of methionine,
(2) a first crystallizing step: a step of introducing carbon dioxide into the reaction solution and precipitating methionine from the reaction solution to obtain first crystals of methionine and a first crystal mother liquor, and
(3) a second crystallizing step: a step in which after obtaining a slurry which contains precipitate containing second crystals of methionine precipitated by adding alcohol to the first crystallization mother liquor and then introducing carbon dioxide, (a) the slurry is subjected to centrifugation to separate the second crystals of methionine and the second crystal mother liquor by a solid-liquid separation at a centrifugal force 100 times or more to 4000 times or less gravity, and (b) precipitate remaining in the obtained second crystal mother liquor is separated with a filter.

2. The method according to claim 1 wherein the precipitate is dissolved in the first crystal mother liquid, and added to the reaction solution.

3. The method according to claim 1 or 2 wherein a portion of the second crystal mother liquor is recycled to the second crystallization step.

4. The method according to any one of claims 1 to 3 wherein the material of the filter is polyphenylene sulfide or polypropylene.

5. A production apparatus comprising
a reaction apparatus in which 5-[2-(methylthio)ethyl]imidazolidine-2,4-dione is hydrolyzed in the presence of an alkali compound to obtain a reaction solution containing an alkali salt of methionine,
a first crystallization apparatus in which carbon dioxide is introduced into the reaction solution to precipitate methionine from the reaction solution, and
a second crystallization apparatus in which alcohol is added to the first crystallization mother liquor which is obtained after separating methionine obtained in the first crystallization apparatus and carbon dioxide is introduced to obtain a slurry containing the precipitated precipitates,
a centrifugal separator for solid-liquid separation of the slurry containing the precipitate at a centrifugal force 100 times or more to 4000 times or less gravity, and
a filter for separating the precipitates remaining in the second crystal mother liquid obtained by a solid-liquid separation with the centrifuge separator.

## Patentansprüche

1. Verfahren zum Produzieren von Methionin, das die folgenden Schritte (1) bis (3) umfasst:
(1) einen Reaktionsschritt: ein Schritt des Hydrolysierens von 5-[2-(Methylthio)ethyl]imidazolidin-2,4-dion in der Gegenwart einer Alkaliverbindung, um eine Reaktionslösung zu erhalten, die ein Alkalisalz von Methionin enthält,
(2) einen ersten Kristallisationsschritt: ein Schritt des Einführens von Kohlendioxid in die Reaktionslösung und des Ausfällens von Methionin aus der Reaktionslösung, um erste Kristalle von Methionin und eine erste Kristallmutterlauge zu erhalten, und
(3) einen zweiten Kristallisationsschritt: einen Schritt, bei dem nach dem Erhalten einer Aufschlämmung, die einen Niederschlag enthält, der zweite Kristalle von Methionin enthält, die durch Zugeben von Alkohol zu der ersten Kristallisierungsmutterlauge und dann Einführen von Kohlendioxid ausgefällt wurden, (a) die Aufschlämmung einer Zentrifugation unterzogen wird, um die zweiten Kristalle von Methionin und die zweite Kristallmutterlauge durch eine Fest-Flüssig-Trennung bei einer Zentrifugalkraft, die das 100-fache oder mehr bis zu dem 4000-fachen oder weniger der Schwerkraft beträgt, zu trennen, und (b) der in der erhaltenen zweiten Kristallmutterlauge verbleibende Niederschlag mit einem Filter abgetrennt wird.

2. Verfahren nach Anspruch 1, wobei der Niederschlag in der ersten Kristallmutterflüssigkeit aufgelöst wird und zu der Reaktionslösung zugegeben wird.

3. Verfahren nach Anspruch 1 oder 2, wobei ein Abschnitt der zweiten Kristallmutterlauge in den zweiten Kristallisationsschritt zurückgeführt wird.

4. Verfahren nach einem der Ansprüche 1 bis 3, wobei das Material des Filters Polyphenylensulfid oder Polypropylen ist.

5. Produktionseinrichtung, umfassend
eine Reaktionseinrichtung, in der 5-[2-(Methylthio)ethyl]imidazolidin-2,4-dion in der Gegenwart einer Alkaliverbindung hydrolysiert wird, um eine Reaktionslösung zu erhalten, die ein Alkalisalz von Methionin enthält,
eine erste Kristallisationseinrichtung, in der Kohlendioxid in die Reaktionslösung eingeführt wird, um Methionin aus der Reaktionslösung auszufällen, und
eine zweite Kristallisationseinrichtung, in der Alkohol zu der ersten Kristallisationsmutterlauge zugegeben wird, die nach dem Trennen des in der ersten Kristallisationseinrichtung erhaltenen Methionins erhalten wird, und Kohlendioxid eingeführt wird, um eine Aufschlämmung zu erhalten, die die ausgefällten Niederschläge enthält,
einen Zentrifugalseparator für die Fest-Flüssig-Trennung von der den Niederschlag enthaltenden Aufschlämmung bei einer Zentrifugalkraft, die das 100-fache oder mehr bis zu dem 4000-fachen oder weniger der Schwerkraft beträgt, und
ein Filter zum Trennen der Niederschläge, die in der zweiten Kristallmutterlauge zurückbleiben, die durch eine Fest-Flüssig-Trennung mit dem Zentrifugalseparator erhalten wird.

## Revendications

1. Procédé pour produire de la méthionine, comprenant les étapes suivantes (1) à (3)
(1) une étape de réaction: une étape d'hydrolysation de 5-[2-(méthylthio)éthyl]imidazolidine-2,4-dione en présence d'un composé alcalin pour obtenir une solution de réaction contenant un sel alcalin de méthionine,
(2) une première étape de cristallisation : une étape d'introduction de dioxyde de carbone dans la solution de réaction et de précipitation de la méthionine provenant de la solution de réaction pour obtenir des premiers cristaux de méthionine et une première liqueur mère cristalline, et
(3) une seconde étape de cristallisation : une étape dans laquelle, après l'obtention d'une bouillie qui contient un précipité contenant des seconds cristaux de méthionine précipités en ajoutant de l'alcool à la première liqueur mère de cristallisation et puis en introduisant du dioxyde de carbone, (a) la bouillie est soumise à une centrifugation pour séparer les seconds cristaux de méthionine et la seconde liqueur mère cristalline par l'intermédiaire d'une séparation solide-liquide à une force centrifuge de 100 fois ou plus à 4 000 fois ou moins la gravité, et (b) un précipité restant dans la seconde liqueur mère cristalline obtenue est séparé avec un filtre.

2. Procédé selon la revendication 1 dans lequel le précipité est dissout dans le premier liquide mère cristallin, et ajouté à la solution de réaction.

3. Procédé selon la revendication 1 ou 2, dans lequel une partie de la seconde liqueur mère cristalline est recyclée vers la seconde étape de cristallisation.

4. Procédé selon l'une quelconque des revendications 1 à 3, dans lequel le matériau du filtre est sulfure de polyphénylène ou du polypropylène.

5. Appareil de production comprenant
un appareil de réaction dans lequel de la 5-[2-(méthylthio)éthyl]imidazolidine-2,4-dione est hydrolysée en présence d'un composé alcalin pour obtenir une solution de réaction contenant un sel alcalin de méthionine,
un premier appareil de cristallisation dans lequel du dioxyde de carbone est introduit dans la solution de réaction pour précipiter de la méthionine provenant de la solution de réaction, et
un second appareil de cristallisation dans lequel de l'alcool est ajouté à la première liqueur mère de cristallisation qui est obtenue après avoir séparé la méthionine obtenue dans le premier appareil de cristallisation, et du dioxyde de carbone est introduit pour obtenir une bouillie contenant les précipités ayant été précipités,
un séparateur centrifuge pour une séparation solide-liquide de la bouillie, contenant le précipité, à une force centrifuge de 100 fois ou plus à 4 000 fois ou moins la gravité, et
un filtre pour séparer les précipités restant dans le second liquide mère cristallin obtenu par l'intermédiaire d'une séparation solide-liquide avec le séparateur centrifuge.
